# EUROPEAN PATENT SPECIFICATION

(11) **EP 4 661 749 B1**
(45) Date of publication and mention of the grant of the patent: **24.06.2026**
(21) Application number: 24705774.8
(22) Date of filing: 09.02.2024
(51) Int. Cl.: A61B 5/11, A61B 5/00

(54) **MOVEMENT DISORDER DETECTION AND ASSESSMENT**
ERKENNUNG UND BEURTEILUNG VON BEWEGUNGSSTÖRUNGEN
DÉTECTION ET ÉVALUATION DE TROUBLE DU MOUVEMENT

(30) Priority: 10.02.2023 US 202363444674 P
(43) Date of publication of application: 17.12.2025
(73) Proprietor: Teva Pharmaceutical Industries Ltd., Tel Aviv 6944020 (IL)
(72) Inventor: REICH, Michael, 6944020 Tel Aviv (IL); GOTLER, Alex, 6944020 Tel Aviv (IL); EFRATI, Itamar, 6944020 Tel Aviv (IL)
(74) Representative: AWA Sweden AB
(86) International application number: PCT/IL2024/050156
(87) International publication number: WO 2024/166115

(56) References cited:
- US-A1- 2019 110 736
- US-A1- 2021 188 291
- US-A1- 2023 027 320

## Description

### CROSS-REFERENCE TO RELATED APPLICATIONS

This application claims priority to U.S. provisional patent application no. 63/444,674, filed February 10, 2023.

### BACKGROUND

Tardive dyskinesia (TD) is a movement disorder that affects the nervous system. For example, TD may cause a range of involuntary repetitive muscle movements in the face, arms, and legs. Typically, a person develops TD by using certain psychiatric drugs for an extended period of time. TD is generally underdiagnosed as patients are typically prescribed psychiatric drugs from a psychiatrist who monitors that patient's response to the drug but is not trained in diagnosing movements disorders caused by the prescribed drug. Further, TD may also be underdiagnosed as trained neurologists are not attentive to the possibility of TD causing the patient's symptoms. US2019/110736 Al discloses a machine-learning-based method for assessing involuntary movements associated with tardive dyskinesia.

For those patients seeking a diagnosis for or being treated for TD, the patient is usually advised to undergo a time consuming Abnormal Involuntary Movement Scale (AIMS) test. A conventional AIMS test may require the patient to visit a trained clinician in person, making the AIMS test even more time consuming. As such, patients may quit seeking a diagnosis or treatment for TD after not receiving a timely TD diagnosis or after not seeing appropriate clinical improvement due to infrequent AIMS testing. Further, the results of an AIMS test may be inconsistent between clinicians, as a portion of the test relies upon at least partially subjective judgments made by the clinician.

### SUMMARY

The present disclosure relates generally to detecting and assessing a movement disorder, and more particularly, to assessing a risk of a patient for tardive dyskinesia (TD). The disclosed technology relates to a method as claimed in claim 1. Preferred embodiments are in accordance with claims 2-15.

The methods described herein may be performed by one or more devices where, for example, each device may include a memory configured to store instructions and one or more processors configured to read the stored instructions. In one or more cases, the disclosed technology relates to a system for assessing risk of a patient for tardive dyskinesia.

### BRIEF DESCRIPTION OF THE DRAWINGS

FIG. 1A illustrates a schematic diagram of an example system environment.
FIG. 1B illustrates a block diagram showing an example of one or more portions of the example system environment of FIG. 1A.
FIG. 2 is a flowchart that illustrates an example performance of assessing a risk of a patient for tardive dyskinesia.
FIG. 3 illustrates an example interface displaying an example prompt to a patient.
FIG. 4 is a block diagram of an example computing device.
FIG. 5 is a diagram of an example facial mask that includes one or more landmarks associated with a patient.

### DETAILED DESCRIPTION

The following discussion omits or only briefly describes conventional features of treatment or diagnostic systems, which are apparent to those skilled in the art. It is noted that various embodiments are described in detail with reference to the drawings, in which like reference numerals represent like parts and assemblies throughout the several views. Reference to various embodiments does not limit the scope of the claims attached hereto. Additionally, any examples set forth in this specification are intended to be non-limiting and merely set forth some of the many possible embodiments for the appended claims. Further, particular features described herein can be used in combination with other described features in each of the various possible combinations and permutations.

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/or "comprising," when used in this specification, specify the presence of stated features, elements, and/or components, but do not preclude the presence or addition of one or more other features, steps, operations, elements, components, and/or groups thereof.

For the diagnosis and assessment of Tardive Dyskinesia (TD), the Abnormal Involuntary Movement Scale (AIMS) is the primary tool utilized by clinicians. The AIMS is a test comprising a combination of items rated on a 5-point anchored scale, including orofacial movements, extremity movements, and truncal movements, as well as global severity of movements as judged by the examiner, in addition to yes-no questions (e.g., graded on a binary scale) concerning dental health. To assess the severity of each of the items assessed on the anchored scale, the clinician instructs the patient to undergo a series of specific activities, such as instructing the patient to protrude their tongue, flex and extend the patient's arms, etc. The 5-point anchored scale is utilized to indicate a severity of involuntary movements of the patient's body. For instance, a movement severity score of zero indicates no involuntary movement, a movement severity score of one indicates minimal or normal involuntary movement, a movement severity score of two indicates mild involuntary movement, a movement severity score of three indicates moderate involuntary movement, and a movement severity score of four indicates severe involuntary movement.

The AIMS can only be performed by appropriately trained clinicians. TD may generally be underdiagnosed or improperly diagnosed, given that TD develops in patients prescribed certain psychiatric drugs for an extended period from clinicians such as psychiatrists or other medical professionals who often are not certified to administer the AIMS. As such, access to an accurate TD diagnosis can be difficult. Even when TD has been diagnosed by a certified clinician, the complexity of the AIMS test can prevent TD patients from obtaining periodic AIMS testing to track and document treatment progress, which can lead to frustration in treatment and subsequent treatment abandonment. Also, as the AIMS involves implicitly subjective determinations, AIMS scoring can differ even between trained clinicians.

As a result, there is a need for a diagnostic tool for easily and uniformly assessing TD risks and symptoms in patients.

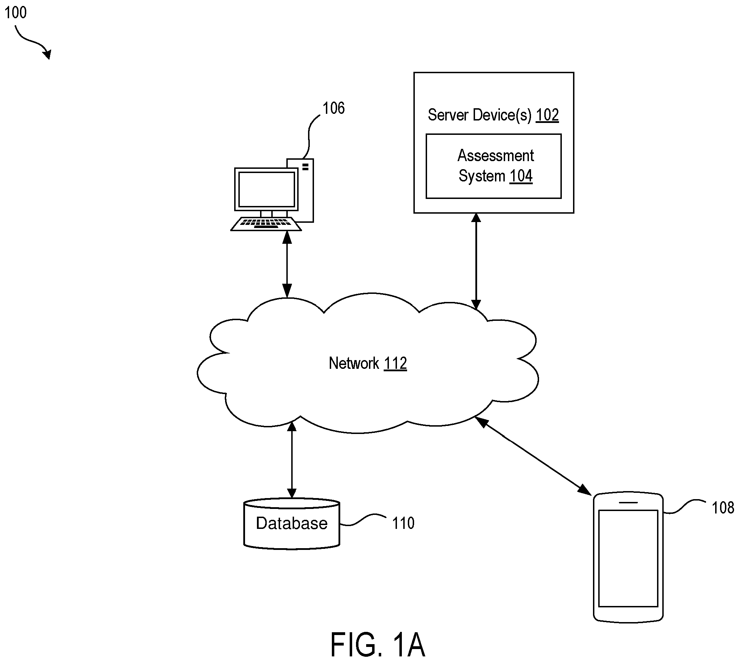

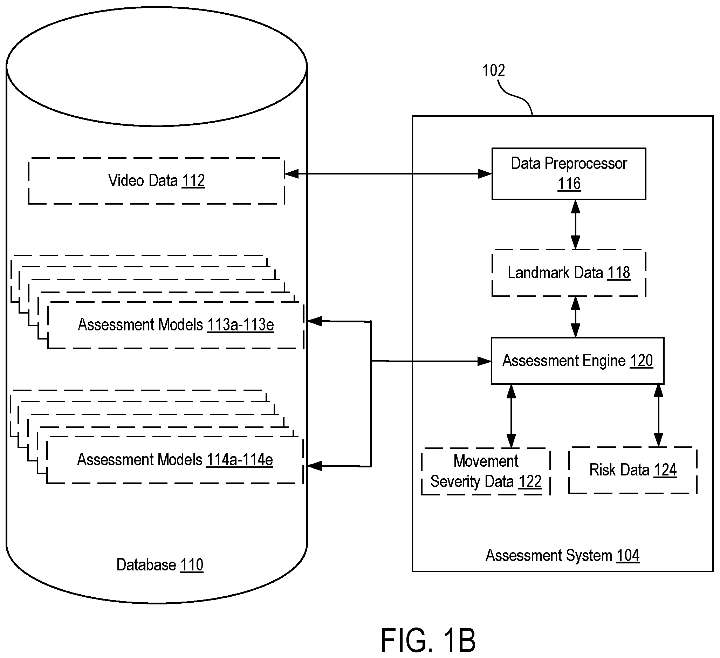

FIG. 1A illustrates a schematic diagram of a system environment (or "environment") 100, for implementing a TD diagnostic system as described herein. As illustrated, the environment 100 includes one or more server device(s) 102 connected to one or more of a device 106 *(e.g.,* a clinician device), a device 108 *(e.g.,* a patient device), and a database 110 via a network 112. FIG. 1B illustrates a block diagram showing an example of one or more portions of the example system environment 100 of FIG. 1A.

As shown in FIG. 1A, the server device(s) 102, the device 106, the device 108, and the database 110 may communicate with each other via the network 112. The network 112 may comprise any suitable network over which computing devices can communicate. The network 112 may include a wired and/or wireless communication network. Example wireless communication networks may be comprised of one or more types of radio frequency (RF) communication signals using one or more wireless communication protocols, such as a cellular communication protocol, a wireless local area network (WLAN) or WIFI communication protocol, and/or another wireless communication protocol. Though FIG. 1A illustrates the components of environment 100 communicating via the network 112, it will be appreciated that the components of environment 100 may communicate directly with each other, for example, bypassing the network 112. For example, the device 108 may communicate directly with the server device(s) 102.

The server device(s) 102 may generate, receive, analyze, store, and/or transmit digital data, such as video data of a patient. In one or more cases, the server device(s) 102 may communicate with the device 106 and the device 108. For example, the server device(s) 102 may send data to the device 106, including video data or other information, such as movement severity scores and/or a total severity score. In another example, the server device(s) 102 may receive input from the user (e.g., the patient) via device 108 or the clinician via device 106. In some cases, the server device(s) 102 may include a distributed collection of servers, in which the server device(s) 102 include a number of server devices distributed across the network 112. In some examples, the distributed server device(s) 102 may be located in the same location or at different physical locations. In other cases, the server device(s) 102 may comprise a content server, an application server, a communication server, a web-hosting server, or another type of server.

In one or more cases, the server device(s) 102, device 106, and/or device 108 may include an assessment system 104 or portions thereof. The assessment system 104 may analyze video data of a patient and identify landmarks of the patient. Further, the assessment system 104 may apply one or more trained machine learning models, such as assessment models 114, to the video data and identified landmarks to determine a spatial arrangement and temporal shift of the identified landmarks. In some cases, the assessment system 104 may identify the landmarks prior to applying the trained machine learning model to the identified landmarks. For instance, the assessment system 104 may utilize one or more image processing techniques (e.g., Haar Cascade technique, integral projection technique, Fisher face technique, elastic graph matching technique, radial basis function network recognition technique, hidden Markov model technique, and other like techniques for facial recognition).

The assessment system 104 may apply the trained machine learning model to video data *(e.g.,* corresponding to one video of the patient or a series of videos of the patient) to determine movement of landmark positions over a period of time. Having determined the spatial arrangement and temporal shift of the identified landmarks, the trained machine learning model, such as assessment models 113a-113e, 114a-114e, may determine one or more movement severity scores indicating a severity of involuntary movements of a patient's body. Each movement severity score may be associated with a distinct *(e.g.,* unique) movement of the patient, such as an upper face movement, tongue movement, mouth and lower face movement, head shaking, and/or head tilting. In some cases, based on at least the movement severity scores, the assessment system 104 generates a total severity score indicating a risk or severity of the analyzed movement disorder. The movement severity score and/or the total severity score may be on the scale used for the AIMS test or may be on a different scale *(e.g.,* one to ten, A to F, *etc.).*

It is noted that the assessment system 104 and processes described herein relate to determining evidence of TD. However, it is noted that the processes for detecting TD described herein are exemplary in nature and are not limited to solely detecting TD. As such, it is understood that the processes described herein may be used to detect and provide treatment for other movement disorders *(e.g.,* Huntingdon's disease).

The assessment system 104, or one or more portions thereof, residing on the device 106 or device 108 may allow for on-device analysis of assessing a risk of the patient for a movement disorder, such as, but not limited to, TD. The assessment system 104, or one or more portions thereof, residing on either the device 106 or device 108 may allow the respective device, such as device 106 or device 108, to assess the risk of a patient for TD or severity of TD for a patient. In one or more cases, the assessment system 104, or one or more portions thereof, may reside on the server device(s) 102, such that the device 106 and/or device 108 may offload certain risk analysis tasks for being performed at the server device(s) 102 and/or request information from the server device(s) 102 to enable the device 106 and/or device 108 to perform as described herein.

In one or more cases, the assessment system 104 operates on a central server, such as the server device(s) 102, and may be utilized by one or more computing electronic devices, such as devices 106 and 108, via an application downloaded from the central server or a third-party application store and executed on the one or more computing electronic devices. In one or more cases, the assessment system 104 may be a software-based program, downloaded from a central server, such as the server device(s) 102, and installed on one or more computing electronic devices, such as devices 106 and 108. In one or more cases, the assessment system 104 may be utilized as a software service provided by a third-party cloud service provider (not shown). In one or more cases, the assessment system 104 may be preinstalled, as software and/or firmware, on the one or more computing electronic devices. In one or more cases, the assessment system 104 may be installed onto the one or more computing electronic devices via an external storage device, such as a universal serial bus (USB) flash drive

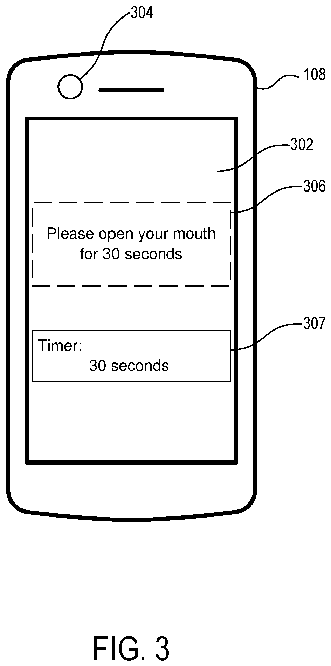

In one or more cases, the device 108 is an electronic computing device, such as a desktop computer, a laptop computer, a tablet computer, a personal digital assistant (PDA), a smart phone, a thin client, or any other electronic device or computing system capable of communicating with the server device(s) 102 through the network 112. The device 108 may be a client to the server device(s) 102. The device 108 can be configured with a camera *(e.g.,* camera 304 illustrated in FIG. 3) or another type of imaging device suitable for acquiring images and video, such as, for example, video of a patient performing an action in response to a prompt from the assessment system 104. In other cases, the device 108 can be any suitable type of mobile device capable of running mobile applications, including smart phones, tablets, slate, or any type of device that runs a mobile operating system. For example, the device 108 may be a mobile device operated by a user, such as a patient, and capable of connecting to a network, such as the network 112, to transmit captured video of the patient. In yet other cases, the device 108 can be any wearable electronic device, such as a head-mounted display, a smartwatch, or the like that is capable of sending, receiving, and processing data.

In one or more cases, the device 108 can include a user interface for providing an end user with the capability to interact with the assessment system 104. A user interface refers to the information (such as graphics, text, and sound) the assessment system 104 presents to a user and the control sequences the user employs to control the assessment system 104 and respond to prompts generated by the assessment system 104. A user interface can be, for example, a keyboard that allows a user to input text, a camera that can recognize user gestures and/or objects, a touchscreen that accepts input from a user via touch of a body part and/or a stylus, or the like. A user may access the assessment system 104 through the user interface to enable the assessment system 104 to operate on the user's device, such as device 108.

In one or more cases, the device 106 includes one or more of the same or similar features as discussed with respect to the device 108. Accordingly, a description of such features is not repeated. In other embodiments, the device 106 can comprise a system including a digital camera or other motion recording device in communication with a separate electronic computing device.

FIG. 1B illustrates a block diagram showing an example of one or more portions of the example system environment 100 of FIG. 1A. The database 110 is configured to store information such as captured video data 112, facial landmark data 118, movement severity data 122, risk data 124, patient information, and the like. Further, the database 110 may be configured to store trained machine learning models, such as, but not limited to assessment models 113a-113e, 114a-114e. It is noted that the database 110 may store information for more than one patient. It is also noted that the information stored within the database 110 may be tagged with an identifier, that correlates each piece of data associated with the patient. Further, information stored within the database 110 may be stored as anonymized data. As described herein, the information stored within the database 110 may be localized on the database 110, on the server device(s) 102, or distributed on the database 110, server device(s) 102, and other data storage repositories and servers. Additionally, although FIG. 1B illustrates assessment models 113a-113e, 114a-114e as being localized on database 110, it should be understood that assessment models 113a-113e, 114a-114e may also be localized on assessment system 104 or distributed across database 110 and assessment system 104.

In one or more cases, the assessment system 104 includes a data preprocessor 116, the features of which may be implemented in hardware and/or software. The data preprocessor 116 may be configured to receive raw signals, such as video data 112, and to process the raw signals to remove invalid data, anomalies, and the like. For example, the data preprocessor 116 may be configured to receive raw signals representing video data 112 captured by camera, such as a camera of the device 106 or 108. The data preprocessor 116 may be configured to process the raw video data 112 to determine facial landmark data 118, which, in some examples, may be used to identify landmarks of a patient. In one or more cases, the data preprocessor 116 may process the raw signals in an online mode, *i.e.,* processing the raw signals as the signals are received, for example, from the camera of the device 108, using one or more data buffers. In one or more other cases, the data preprocessor 116 may process the raw signals in an offline mode, i.e., processing raw signals retrieved from a data storage repository, such as the database 110. For the cases in which the server device(s) 102 utilizes a distributed computing system, the data preprocessor 116 utilizes the distributed computing components of the server device(s) 102 to process the raw signals in parallel.

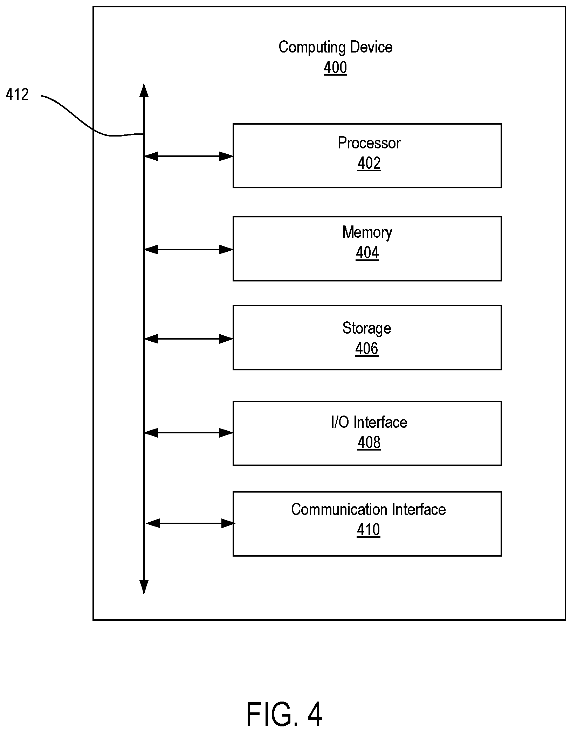

In one or more cases, the assessment system 104 includes an assessment engine 120, which may be implemented in hardware. In one or more other cases, the assessment engine 120 may be implemented as an executable program maintained in a tangible, non-transitory memory, such as memory (e.g., memory 404 of FIG. 4), which may be executed by one or processors *(e.g.,* such as processor 402 of FIG. 4). As further described herein, the assessment engine 120 may be configured to implement the assessment models 113a-113e, 114a-114e to generate movement severity data 122 and risk data 124 and determine a risk of the patient for TD or severity of TD symptoms.

The first trained machine learning models 113a-113e may be configured to determine whether a particular category of facial movement associated with TD (e.g., upper face movement, tongue movement, mouth and lower face movement, head shaking, and head tilting) is present within the video data. In some examples, the first trained machine learning models 113a-113e may determine whether a category of facial movement associated with TD based on facial landmark data 118. In some examples, the output of each of the first trained machine learning models 113a-113e can be a binary indication of whether a particular category of facial movement associated with TD is present within a video segment. In other examples, the output of each of the first trained machine learning models 113a-113e can comprise a percent chance of whether a particular category of facial movement associated with TD is present within the video segment. As such, the first trained machine learning models 113a-113e can produce a movement indicator *(e.g.,* such as a numerical indication) that indicates of whether a particular category of facial movement associated with TD is present within the video segment. The assessment system 104 may accordingly apply the trained machine learning models 113a-113e to a reduced amount of data, i.e., the facial landmark data 118 that is relevant to the category of facial movement corresponding to a particular algorithm 113a-113e.

The assessment engine 120 may apply the assessment models 114a-114e to the movement indicators produced by the assessment models 113a-113e to generate movement severity data. The movement severity data (e.g., a movement severity score) may indicate a severity of movement. In some examples, the movement severity data may be specific for each of a plurality of different categories of facial movements. The movement severity score may be scored on a scale that indicates a severity of involuntary movements of the patient's body. In one example, the scale corresponds to the 0-4 scale utilized in the AIMS test. For instance, a movement severity score of zero may indicate no involuntary movement, a movement severity score of one may indicate minimal or normal involuntary movement, a movement severity score of two may indicate mild involuntary movement, a movement severity score of three may indicate moderate involuntary movement, and a movement severity score of four may indicate severe involuntary movement. However, it is contemplated that other scales may be utilized (0-1, 0-10, 0-100, etc.).

The assessment system 104, and preferably the assessment engine 120, may apply a first trained machine learning model 113a-113e to facial landmark data within the video data to generate movement indicators of a category of facial movement. Each of the first trained machine learning models 113a-113e may be unique to a specific category of facial movement (e.g., model 113a for analysis of blinking, model 113b for analysis of head tilt, etc.). The assessment system 104, and preferably the assessment engine 120, may apply a corresponding second trained machine learning model 114a-114e (e.g., model 114a for analysis of blinking, model 114b for analysis of head tilt, etc.) to the movement indicators produced by a corresponding first trained machine learning models 113a-113e to generate movement severity data based on an entire assessment session for a particular patient. As such, the assessment engine may utilize two trained machine learning models unique to each of the categories of facial movement (e.g., models 113a, 114a for analysis of blinking, models 113b, 114b for analysis of head tilt, etc.) to generate movement severity data for the patient.

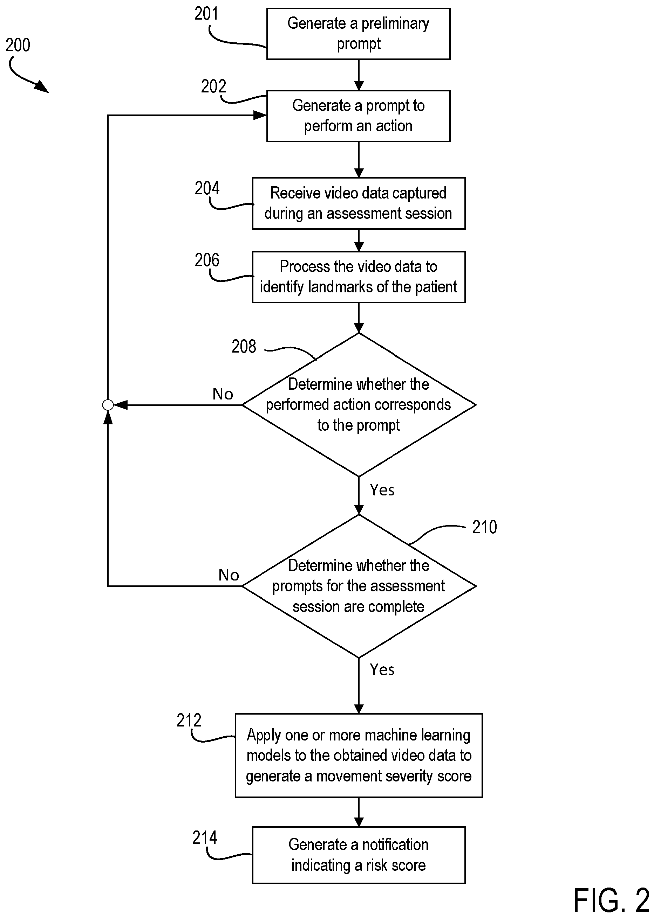

FIG. 2 is a flowchart that illustrates an example procedure 200 of assessing a risk of a patient for TD. An assessment system, such as the assessment system 104, may perform one or more steps of the procedure 200. Although described primarily in the context of the procedure being performed by the assessment system 104, in some examples, one or more of the device 106 and/or the device 108 may perform any combination of the steps of the procedure 200 *(e.g.,* in combination with the assessment system 104). The risk of TD may be assessed in a variety of locations. For instance, the patient may perform the assessment at home (e.g., via device 108). In another instance, the patient may perform the assessment while at a clinician's office *(e.g.,* via device 106). In one or more cases, to access the assessment system 104, the patient may login to an assessment portal, via, for example, but not limited to a telehealth application. It is noted that in the example provided herein, the patient may perform the assessment via device 108. However, it should be understood that one, more, or all of the processes discussed herein for performing the assessment via device 108 may also be implemented via device 106.

The assessment system 104 may generate one or more preliminary prompts to the respective device, for example, device 108, to prepare the patient to begin the assessment (201). The device 108 may display the preliminary prompts on a user interface of the device 108 (*e.g.,* via the user interface 302). For example, one prompt may instruct the patient to remove any objects *(e.g.,* gum or candy) from the patient's mouth. In another example, another prompt may instruct the patient to begin the assessment while sitting in a chair that is hard, firm, and one without arms. In another example, another prompt may instruct the patient to remove their shoes and socks. In yet another example, another prompt may instruct the patient to position the device 108 such that the device 108 is in a particular position and a camera 304 of the device 108 can capture video data of the patient. For example, the prompt may specify that the camera 304 of the device 108 should be placed at a specific distance from the patient and/or a specific height relative to the patient. Additionally, the prompt may specify that the camera 304 of the device 108 should be in placed in a stable, stationary position. One example of a prompt may be a notification that is generated via graphical user interface (GUI) on a display device of one or more devices described herein, such as a notification provided via a display of the device 106 and/or the device 108.

A prompt to perform an action is generated (202), preferably by the assessment system 104. For example, the assessment system 104 may provide a prompt to the device 108, which displays the prompt on the user interface. For example, the displayed prompt 306 may instruct the patient to "Please open your mouth for 30 seconds", for example, as illustrated in FIG. 3. In one or more cases, the user interface 302 may display a timer *(e.g.,* the timer 307) that displays a time corresponding to the displayed prompt *(e.g.,* the displayed prompt 306). When capturing video data of the patient, the timer may provide an indication of the length of time remaining to complete the prompted instruction. For example, when the camera begins capturing video of the patient with an open mouth, the timer may countdown from a predefined time period, such as 30 seconds.

The assessment system 104 may generate a prompt to perform a scripted action. A prompt for a scripted action may include, for example, but not limited to, an instruction for the patient to perform a series of actions. For example, a prompt may instruct the patient to open the patient's mouth for a first period of time, briefly close the patient's mouth at the expiration of the first period of time, and subsequently open the patient's mouth for a second period of time. In another example, a prompt may instruct the patient to open the patient's mouth and protrude the patient's tongue twice. In other examples, a prompt for a scripted action may include, for example, but not limited to, an instruction for the patient to perform a singular activity.

In addition to or in the alternative to providing a generate to perform a scripted action, the assessment system 104 may generate a prompt to perform an unscripted action. A prompt for an unscripted action may include, for example, but not limited to, an instruction for the patient to stare at the display screen of the device 108 for a certain period of time.

Further, in other examples, the prompt may be for the patient to stay still *(e.g.,* not move) for a duration of time, for instance, while the patient maintains their head, face, and/or mouth in a particular orientation.

Video data captured during an assessment session is received (204), such as by the assessment system 104. For example, the assessment system 104 may capture video data during the assessment session via a camera of the device 108 *(e.g.,* camera 304). For instance, during the assessment session, the camera may record video of the patient at rest or performing a bodily movement in response to the prompt displayed on the user interface *(e.g.,* the user interface 302). The device 108 may provide the captured video as video data to the assessment system 104. In one or more cases, the device 108 may begin recording video when the assessment session commences. In one or more other cases, the device 108 may begin recording video in response to receiving a prompt from the assessment system 104. In one or more cases, the assessment system 104 may store the video data *(e.g.,* video data 112) in the database 110. The video data can be captured by a camera, such as a camera of device 108, that is focused on a particular region of the patient's body. For example, the camera by be configured to capture video data of the patient's facial region.

In one or more cases, the video data is processed to identify landmarks of the patient (206), for example, by the assessment system 104. For example, the assessment system 104, and for example the data preprocessor 116, may extract and analyze the video data, such as video data 112, to identify landmarks *(e.g.,* facial landmark data and/or other bodily landmarks, such as landmarks of the hands, feet, or trunk) of the patient by utilizing one or more image processing techniques *(e.g.,* dlib facial recognition, Haar Cascade technique, Fisherface method, elastic graph matching technique, and other like techniques for facial recognition).

In the example of dlib facial recognition, the assessment system 104 may receive video data of the patient, and the video data may include a plurality of frames of the patient's face. The assessment system 104 may include a detector to identify the faces within each frame of the video data, a shape predictor to identify landmarks 117 within the video data *(e.g.,* to precisely localize the face), and/or a face recognition model (e.g., such as dlib facial recognition). The assessment system 104 may map frames of the video data that include an image of the patient's face to a multi-dimensional vector space *(e.g.,* a 128-dimensional vector space). In some examples, the assessment system 104 may be configured to identify a bounding box for the patient's face *(e.g.,* identify a set of landmarks 117 associated with the patient's face within each frame of the video data). The assessment system 104 can perform face recognition by mapping landmarks associated with the patient's face to the multi-dimensional vector space and then comparing the Euclidean distance of the identified landmarks to a distance threshold *(e.g.,* a distance threshold of 0.6) to ensure that the patient's face is being accurately and consistently identified across frames of the video data. In some examples, the assessment system 104 may determine an accuracy of close to 100% *(e.g.,* 99.38%) on the standard Labeled Faces in the Wild (LFW) face recognition benchmark.

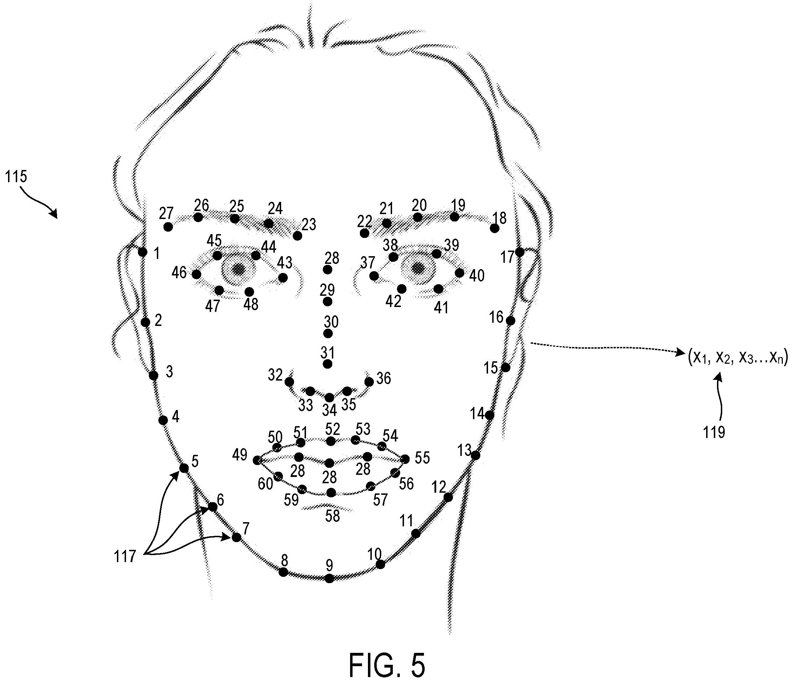

Referring to FIG. 5, in some embodiments, the data preprocessor 116 may extract and analyze the video data 112 and output a facial mask 115 comprising a plurality of unique, uniquely labeled landmarks 117 *(e.g.,* numbered 1-60 in FIG. 5, as an example). In such cases, the data preprocessor 116 may extract and analyze the video data 112 per each frame of video to determine a facial mask 115, and likewise constituent landmarks 117 of the patient. Such landmarks 117 may correspond to physical landmarks on the patient's face, such as the facial outline, eyes, nose, lips, tongue, a nasion, an inion, a lateral canthus, an external auditory meatus (e.g., ear attachment point), one or more preauricular points of the human subject, and the like. For example, the points representing the landmarks 117 may be indicated as coordinates *(e.g.,* X and Y coordinates for standard facial datasets, or X, Y, and Z coordinates for three-dimensional facial datasets) represented in units of pixels and/or a gradient of pixels corresponding to the predicted regions or landmarks. Using the aforementioned image processing techniques, the assessment system 104 *(e.g.,* the data preprocessor 116) can extract and store facial landmark data 118 corresponding to each of the landmarks 117 identified in one or more frames of the video data 112 as a data array and/or data table. For example, facial landmark data 118 corresponding to a particular landmark 117 may be in the form of data array 119. Facial landmark data 118 can include landmark identifier, landmark coordinate position, frame source identifier, video source identifier, patient *(e.g.,* anonymized) identifier, etc. The assessment system 104 may store the identified landmarks 117 of the patient as facial landmark data 118 in the database 110. Finally, it should be appreciated that the landmarks 117 illustrated in FIG. 5 are a subset of the actual number of landmarks that can be identified by the data preprocessor 116 of the assessment system 104.

It is noted that the examples described herein relate to the assessment system 104 processing the captured video data 112 during the assessment session. However, it should be understood that in other examples, the assessment system 104 may obtain the captured video data 112 and store the video data 112 in the database 110 during the assessment session. Further, subsequent to providing one or more prompts to the patient and capturing video of the patient performing bodily movements in response to the respective prompt, the assessment system 104 may retrieve the stored video data 112 and process the video data 112 after the patient completed the examination procedure.

The assessment system 104 may analyze the landmarks 117 to determine whether the performed action of the patient corresponds to the prompt *(e.g.,* the prompt generated at 202) (208). In one or more cases, the assessment system 104 may be preprogrammed to associate one or more the identified landmarks 117 with each prompt. For instance, the assessment system 104 may be preprogrammed to associate the prompt "Please open your mouth for 30 seconds" with a first subset of the landmarks *(e.g.,* landmarks labeled 4-14), and the prompt "Please open your mouth and protrude your tongue" with a second subset of the landmarks 118 *(e.g.,* landmarks labeled 49-60). In some instances, for example, by associating a particular subset of the identified landmarks 117 with a prompt, the assessment system 104 may analyze those landmarks 117 to determine whether the performed action corresponds to the prompt (208). For example, in response to providing the patient with a prompt "Please open your mouth for 30 seconds," the assessment system 104 may analyze the first subset of landmarks 117 to determine the spatial arrangement and temporal shifts in those particular landmarks 117.

The assessment system 104 may determine that the performed action does not correspond to the prompt based on the movement in the spatial arrangement of the corresponding landmarks 117. For example, the assessment system 104 may determine that none of the landmarks 117 associated with the patient's cheeks, jaw, lips, and/or tongue move (*i.e.,* an indication that the patient did not open the patient's mouth) in the captured video data 112. In other cases, the assessment system 104 may determine that the performed action does not correspond to the prompt if a positional displacement of spatial arrangement of the corresponding landmarks 117 does not exceed a threshold value. For example, the assessment system 104 may detect a slight movement in the patient's lips and jaw based on the spatial arrangement and shifting of the associated landmarks 117 in a series of frames from the captured video data 112. However, the assessment system 104 may determine that the spatial arrangement and shift of the associated landmarks 117 did not shift beyond a predetermined threshold value *(i.e.,* an indication that the patient did not open the patient's mouth enough to analyze the landmarks associated with the patient's tongue). As such, the assessment system 104 may determine that the performed action does not correspond to the provided prompt.

The assessment system 104 may determine that the performed action corresponds to the provided prompt by applying one or more classifiers associated with the action of the prompt to at least a portion of the temporal shifts of the identified landmarks. In some cases, each classifier may correspond to one or more orofacial movements of the patient. The assessment system 104 may select one or more orofacial movements of the respective classifiers and compare the selected orofacial movements to the temporal shifts of the identified landmarks. In one or more cases, the assessment system 104 may determine that the patient performed the correct action based on the temporal shifts of the identified landmarks being correctly associated with the selected orofacial movements.

For the cases in which it is determined that the performed action does not correspond to the prompt (208:NO), the assessment system 104 provides the same prompt to the device 108 (202), which displays the prompt on the user interface 302. It is noted that in some cases in which the assessment system 104 detects that the patient is not performing the action corresponding to the prompt, the assessment system 104 may issue another prompt or indication to display on the user interface 302 that further directs the patient to perform the action corresponding to the prompt.

The assessment system 104 may determine that the performed action corresponds to the prompt if a positional displacement of spatial arrangement of the landmarks exceeds a threshold value. For example, the assessment system 104 may detect a movement in the patient's lips and jaw based on the spatial arrangement and shifting of the associated landmarks in the captured video data 112. Further, the assessment system 104 may determine that the spatial arrangement and shift of the associated landmarks shifted beyond a predetermined threshold value (e.g., an indication that the patient opened the patient's mouth enough to analyze the landmarks associated with the patient's tongue). As such, the assessment system 104 may determine that the performed action corresponds to the provided prompt.

For the cases in which it is determined that the performed action does correspond to the prompt (208:YES), a determination is made as to whether the prompts for the assessment session are complete (210), preferably by the assessment system 104. For example, an assessment session for certain movement disorders may include a patient responding to a series of prompts to perform respective actions. In response to the patient performing the action corresponding to the provided prompts and the assessment system 104 generating the corresponding movement severity scores, the assessment system 104 may determine whether the prompts for the assessment session are complete. Further, it should be appreciated that in some examples, the system may not determine whether the performed action corresponds to the prompt *(e.g.,* 208 may be omitted from the procedure 200).

For the cases in which it is determined that the prompts for the assessment session are not complete (210:NO), the assessment system 104 provides another prompt to perform an action, as described with respect to 202. For the cases in which it is determined that the prompts for the assessment session are complete (210:YES), the assessment system 104 may apply one or more machine learning models to the obtained video data to generated one or more movement severity scores (212). For instance, upon completion of the patient assessment, one or more trained machine learning models 113a-113e, 114a-114e are applied to the facial landmark data 118 corresponding to the landmarks 117 identified in the obtained video data 112 to generate one or more movement severity scores, preferably by the assessment system 104.

In some examples, the assessment system 104 may individually assess movement severity scores for different categories of facial movement associated with TD symptoms. In such an embodiment, the assessment system 104 may apply one or more trained machine learning models to the facial landmark data 118 to determine a first severity score corresponding a first category of facial movement associated with TD, and one or more different trained machine learning models to the facial landmark data 118 to determine a second severity score corresponding to a second category of facial movement associated with TD. For example, the unique categories of facial movement can include upper face movement, tongue movement, mouth and lower face movement, head shaking, and head tilting, though other unique categories are contemplated.

For example, upon determining that the prompts for performing the assessment session are complete, the assessment system 104 may divide the video data 112 and/or corresponding facial landmark data 118 from a particular assessment session into a plurality of data subsets corresponding to video segments of a particular length less than the length of the overall assessment session. For example, the assessment system 104 may divide the video data 112 and/or the corresponding facial landmark data 118 into subsets corresponding to video segments of 1-30 seconds, 1-20 seconds, or 1-10 seconds. In a particular embodiment, the assessment system 104 may divide the video data 112 and/or the corresponding facial landmark data 118 into subsets corresponding to video segments of 4 seconds. As such, each data subset comprises the facial landmark data 118 associated with each landmark 117 for a particular segment of the assessment session. In one embodiment, the data subsets comprise sequential, non-overlapping segments of the video data 112. In another embodiment, the data subsets comprise sequential, overlapping segments of the video data 112. For example, in one embodiment each sequential data subset corresponding to a 4 second segment of the assessment session may represent a 2 second overlap of the immediately preceding and subsequent 4 second segments.

The assessment engine 120 can then apply a first trained machine learning model (e.g., such as one or more of assessment models 113a-113e) to the facial landmark data 118 comprising each data subset. Each of the first trained machine learning models 113a-113e is configured to determine from the facial landmark data 118 corresponding to each data subset whether a particular category of facial movement associated with TD *(e.g.,* upper face movement, tongue movement, mouth and lower face movement, head shaking, and head tilting) is present within the video segment for the data subset. Such determination can be in the form of a movement indicator, such as a numerical indication. In one embodiment, the output of each of the first trained machine learning models 113a-113e can be a binary indication of whether a particular category of facial movement associated with TD is present within the video segment for the data subset. In another embodiment, the output of each of the first trained machine learning models 113a-113e can comprise a percent chance of whether a particular category of facial movement associated with TD is present within the video segment for the data subset. As such, the first trained machine learning models 113a-113e can produce a numerical indication of whether a particular category of facial movement associated with TD is present within the video segment associated with each data subset to the facial landmark data 118 of each data subset. For example, the first trained machine learning model 113a can produce a numerical indication for each data subset of whether blinking is present, the second trained machine learning model 113b can produce a numerical indication for each data subset of whether tongue movement is present, etc.

It should be appreciated that each of the first trained machine learning models 113a-113e may apply the same or different machine learning algorithms *(e.g.,* any of those described herein). For instance, the machine learning algorithm for each of the first trained machine learning models 113a-113e may be selected based on which machine learning algorithm is most fit to assess the particular category of facial movement that the trained machine learning models 113a-113e is configured to detect.

In one embodiment, all facial landmark data 118 for each of the data subsets can be provided to the assessment models 113a-113e to determine whether facial movement associated with TD are present. Alternatively, each respective assessment model 113a-113e may only be provided with the facial landmark data 118 associated with the corresponding category of facial movement. Such selective feeding of data to the assessment models 113a-113e may be done for statistical reasons, such as to avoid overfitting. For this reason, the assessment system 104 may be preprogrammed with correlations between each landmark 117 and the particular category (or categories) of facial movement they are relevant to. The assessment system 104 may accordingly apply the trained machine learning models 113a-113e to a reduced amount of data, *i.e.,* the facial landmark data 118 that is relevant to the category of facial movement corresponding to a particular algorithm 113a-113e. For example, for a trained model 113a analyzing blinking, the assessment system 104 may filter out facial landmark data 118 corresponding to landmarks 117 not relevant to blinking, and only provide the facial landmark data 118 corresponding to the landmarks 117 relevant to blinking to the trained model 113a. In other cases, the assessment system 104 may apply the trained machine learning models to all facial landmark data 118 comprising landmarks identified in the obtained video data.

After the assessment engine has applied the trained models 113a-113e to the facial landmark data 118, the assessment engine 120 may apply the assessment models 114a-114e to the movement indicators *(e.g.,* numerical indications) produced by the assessment models 113a-113e that correspond to respective data subsets to generate movement severity data 122. Specifically, the movement severity data comprises a movement severity score, *i.e.,* a score corresponding to the severity of facial movement in a patient. In some cases, the assessment system 104, and preferably the assessment engine 120, may apply a first trained machine learning 113a-113e model to the facial landmark data 118 for the data subsets as described above to generate numerical indications of a category of facial movement within a particular data subset, and a corresponding second trained machine learning model 114a-114e to the numerical indications for each data subset produced by the first trained machine learning models 113a-113e to generate movement severity data 122 for each of the one or more categories of facial movement based on an entire assessment session for a particular patient. As such, in its analysis of the facial landmark data 118 for each of the categories of facial movement for a particular patient, the assessment engine 120 may utilize two trained machine learning models unique to each of the categories of facial movement *(e.g.,* models 113a, 114a for analysis of blinking, models 113b, 114b for analysis of head tilt, etc.).

The movement severity data 122 *(i.e.,* movement severity score) for each of the categories of facial movement may indicate a severity of movement. The movement severity score may be scored on a scale that indicates a severity of involuntary movements of the patient's body. In one example, the scale corresponds to the 0-4 scale utilized in the AIMS test. For instance, a movement severity score of zero may indicate no involuntary movement. In another instance, a movement severity score of one may indicate minimal or normal involuntary movement. In another instance, a movement severity score of two may indicate mild involuntary movement. In another instance, a movement severity score of three may indicate moderate involuntary movement. In another instance, a movement severity score of four may indicate severe involuntary movement. However, it is contemplated that other scales may be utilized (0-1, 0-10, 0-100, etc.).

After each of the second trained machine learning models 114a-114e has produced a respective movement severity score for the corresponding category of facial movement, the assessment system 104 may calculate a single total severity score based on each of the movement severity scores. The total severity score may be a composite score incorporating each of the movement severity scores and representative of an overall severity of involuntary facial movements for a particular patient. For example, the total severity score may be determined on a finite scale so as to provide a normalized metric for comparing involuntary facial movement severity between patients, or for a particular patient at different points in time. In one embodiment, the total severity score may represent an average of the movement severity scores produced by second trained machine learning models 114a-114e. It is also contemplated that the total severity score may comprise weighted average and/or other calculation based on the individual movement severity scores. In a particular embodiment, the total severity score may correspond to an AIMS score.

In one or more cases, a notification indicating one or more of the movement severity scores and/or the total severity score is generated (214), preferably by the assessment system 104. In one or more other cases, the assessment system 104 may provide the clinician or prescribing practitioner of the psychiatric drug with the notification indicating the total severity score. In one or more cases, the assessment system 104 may flag the movement severity scores and/or the total severity score as indicating evidence of TD. For instance, if the assessment system 104 determines that the total severity score exceeds a threshold value, the assessment system 104 determines that there is evidence of TD. In another instance, if the assessment system 104 determines that the total severity score exceeds a threshold value, the assessment system 104 provides a recommendation to refer the patient to a trained clinician.

The assessment system 104 may store the movement severity score in the database 110. Further, the assessment system 104 may store the movement severity score as anonymized data in the database 110. One or more subsequent movement severity scores for the patient as provided by a clinician can also be provided to and stored within the database 110, such that subsequently the movement severity score and video data for the patient and the clinician-provided AIMS score can be included within the training data for further training the machine learning models, wherein an exemplary process for training the machine learning model is described below.

The assessment system 104 may be configured to determine a progression of the movement disorder over a period of time. Upon a TD diagnosis, a clinician can prescribe a chemical or biologic medication for the treatment of TD. For example, the clinician can prescribe a treatment comprising deutetrabenazine, valbenazine, tetrabenazine, clonazepam, and/or botulinum toxin.

Further provided herein, are methods useful in treating TD. In some examples, the assessment system 104 may be configured to determine a TD total severity score as described above to inform the creation, maintenance, and/or revisions of a medical treatment plan for the patient, such as administering to a patient in need of a pharmaceutical composition comprising a pharmacologically active agent described herein, in an effective amount to treat TD. As used herein, the terms "method of treatment" or "therapy" (as well as different forms thereof) in relation to tardive dyskinesia, include preventative (e.g., prophylactic), curative, or palliative treatment. As used herein, the term "treating" includes alleviating or reducing at least one adverse or negative effect or symptom of tardive dyskinesia. The term "administering" means providing to a patient the pharmaceutical composition or dosage form (used interchangeably herein). As used herein, the terms "compound", "drug", "pharmacologically active agent", "active agent", or "medicament" are used interchangeably herein to refer to a compound or compounds or composition of matter which, when administered to a subject (human or animal) induces a desired pharmacological and/or physiologic effect by local and/or systemic action. Possible pharmacologically active agent can be selected from tetrabenazine, deutetrabenazine, valbenazine, deuvalbenazine, clonazepam and/or botulinum toxin. One preferred active agent disclosed herein is deutetrabenazine. "Deutetrabenazine" or "deu-TBZ" is a selectively deuterium-substituted, stable, non-radioactive isotopic form of tetrabenazine in which the six hydrogen atoms on the two O-linked methyl groups have been replaced with deuterium atoms (i.e. -OCD3 rather than -OCH3 moieties).

A vesicular monoamine transporter 2 (VMAT2) inhibitor may be prescribed for treating uncontrolled, involuntary movements associated with movement disorder, such as TD. The VMAT2 inhibitor may include, for example, deutetrabenazine (e.g., Austedo^{®} or Austedo^{®} XR), tetrabenazine, and/or valbenazine. In some examples, the total daily dose of the VMAT2 inhibitor can be administered on a once daily basis (qd) or on a twice daily basis (bid). In some examples, the total daily dose of the VMAT2 inhibitor is 6 mg, or 12 mg, or 18 mg, or 24 mg, or 30 mg, or 36 mg, or 42 mg or 48 mg of the VMAT2 inhibitor. The daily amount of drugs may require periodic reevaluation from clinicians, for example, to update the patient's titration schedule. For instance, a patient may receive a package of VMAT2 inhibitor pills having different daily amounts, also referred to as a titration kit. For example, the VMAT2 inhibitor pills may be available in pills with different strengths, such as a 6 mg pill, a 12 pill, and/or a 24 mg pill. A clinician may prescribe the patient with an initial daily amount of a VMAT2 inhibitor. The daily amount may be based on the results of an AIMS test, for example, in combination with other factors associated with the patient. In an embodiment, the daily amount may be based, at least in part, on the total severity score determined by the assessment system 104 as described above. The titration kit may comprise a supply of VMAT2 inhibitor pills for a predetermined period of time *(e.g.,* four weeks). The daily amount of VMAT2 inhibitor may progressively increase throughout the titration kit. For example, the daily amount of VMAT2 inhibitor may increase in a systematic, stepwise manner *(e.g.,* 6 mg/day for week 1, 18 mg/day for week 2, 24 mg/day for week 3, 30 mg/day for week 4, etc.).

The accurate diagnosis and assessment of TD for the patient may require periodic review and assessment of the patient's conditions *(e.g.,* such as through the use of AIMS tests). In an embodiment, the assessment system 104 *(e.g.,* the movement severity scores and/or total severity score generated by the assessment system 104) may be used to flag patients for further assessment for potential TD diagnosis by a trained condition. The patient's clinician may determine an initial daily amount and/or increase the daily amount for the patient after receiving and analyzing a latest periodic evaluation and/or based on a predetermined schedule. The assessment system 104 *(e.g.,* the movement severity scores and/or total severity score generated by the assessment system 104) may be used to determine the titration process for the patient and/or determine the daily amount for the patient to take during any particular interval of treatment *(e.g.,* the initial daily amount). As such, the assessment system 104 may be configured to adjust the patient's dosing schedule and/or dosing amount based on the movement severity scores generated by the assessment system 104. In using the assessment system 104 as an aid in dosing selection and titration, both the clinician and patient are provided with an objective tool that standardizes and expedites the TD assessment process. Examples of titration regimens for the treatment of TD are described in PCT Publication No. WO 2016/0144901, and U.S. Patent Publication No. US 2016/0287574.

In one or more cases, in conjunction with a regimen for the treatment of TD, the patient may utilize the assessment system 104 to perform multiple assessments (e.g., a second assessment) during a subsequent time period to determine a progression of the movement disorder over a period of time. The second assessment may be performed in a same or similar manner as described with the example procedure 200 of FIG. 2. As such, a description of such features is not repeated. As a result of the second assessment, the assessment system 104 may generate a second total severity score. In one or more cases, additional movement severity scores may be obtained as described herein. In one or more cases, the assessment system 104 may compare the second total severity score to the previous total severity score to determine a state of the movement disorder of the patient. For instance, for the cases in which the assessment system 104 determines that the second total severity score is less than the previous total severity score, the assessment system 104 may determine that the severity of the movement disorder is regressing. In another instance, for the cases in which the assessment system 104 determines that the second total severity score is greater than the previous total severity score, the assessment system 104 may determine that the severity of the movement disorder is progressing. In one or more cases, the assessment system 104 may provide a notification to the clinician and/or patient via the device 106 and/or device 108 recommending a reevaluation or adjustment of the currently prescribed treatment regimen.

The assessment system 104 may be configured to determine a progression of a severity of the movement disorder and may generate and provide a notification corresponding to the progression of the severity of the movement disorder, for example, in conjunction with the regimen for the treatment of TD and subsequent assessments that generate corresponding total severity scores. For example, to begin treatment of TD, a patient be administered a total daily dose of 6mg of pharmacologically active agent *(e.g.,* deutetrabenazine) based on an initial total severity score. At a subsequent time *(e.g.,* a week from when the patient performed the first assessment and the assessment system 104 generated the initial total severity score), the patient may perform the second assessment in a same or similar manner as described with the example procedure 200 of FIG. 2 and generate the second total severity score. In one or more cases, additional movement severity scores may be obtained as described herein. In one or more cases, the assessment system 104 or a clinician may compare the second total severity score to the previous total severity score to determine a state of the movement disorder of the patient (*i.e., a* progression of a severity of the movement disorder), whether a current treatment is effective or non-effective based on the state of the movement disorder, and/or whether the patient is failing to follow the treatment regimen. For example, the assessment system 104 and/or a clinician may determine that the second total severity score is greater than the previous total severity score *(e.g.,* the initial total severity score), and thus, the severity of the movement disorder is progressing. Further, the assessment system 104 may provide a notification (e.g., via displaying the notification on an interface of a device, such as device 108) based on the determination that the severity of the movement disorder is progressing indicating a likelihood that the current treatment is no longer effective. The assessment system 104 may provide the state of the movement disorder such that a clinician may determine as to whether the regimen for the treatment of TD should be altered. The patient may continue performing assessments at intervals, such as, but not limited to, weekly intervals, such that the assessment system 104 may generate additional total severity scores and determine a state of the movement disorder. By providing an automated and objective method for repeatedly assessing TD severity and progression, patient adherence can be improved through increased efficiency of TD severity assessment, as well as discrete metrics for observing TD improvement during a treatment regimen.

The machine learning model, such as the assessment models 113a-113e, 114a-114e, may be trained using training data that includes video data from a plurality of patients and total severity scores associated with each patient of the plurality of patients. In some examples, the plurality of trained machine learning models are trained using training data that includes facial landmark data extracted from videos from a plurality of patients with a positive TD diagnosis, Abnormal Involuntary Movement Scale (AIMS) scores associated with each patient of the plurality of patients, and/or labels corresponding to each of the videos indicating a presence of a plurality of distinct category of facial movement.

The video data *(e.g.,* the video data for each patient) may be divided into a plurality of data subsets corresponding to video segments of a particular length less than the length of the overall assessment session. For example, the video data may be divided into subsets corresponding to video segments of 1-30 seconds, 1-20 seconds, or 1-10 seconds. In a particular embodiment, the video data may be divided into subsets corresponding to video segments of 4 seconds. As such, each data subset may include facial landmark data associated with each landmark. In one embodiment, the data subsets comprise sequential, non-overlapping segments of the video data. In another embodiment, the data subsets comprise overlapping segments of the video data. For example, in one embodiment each sequential data subset corresponding to a 4 second segment of the assessment session may represent a 2 second overlap of the immediately preceding and subsequent 4 second segments.

The training data may include only those data subsets where a movement of relevant facial landmark data was detected *(e.g.,* by a physician, by a technician, and/or using a facial recognition tool). For example, the training data may include the video segments *(e.g.,* only the video segments) where a particular category of facial movement associated with TD *(e.g.,* upper face movement, tongue movement, mouth and lower face movement, head shaking, and head tilting) is present within the video segment. Further, in some examples the training data may include *(e.g.,* only include) the facial landmark data associated with the corresponding category of facial movement from each video segment. Such selective feeding of data into the training data may be done for statistical reasons, such as to avoid overfitting. The training data may include correlations between each landmark and the particular category (or categories) of facial movement they are relevant to. Further, in some examples, the training data may include a reduced amount of data, *i.e.,* the facial landmark data that is relevant to the category of facial movement. For example, the training data used to analyze blinking may not include *(e.g.,* have filtered out) the facial landmark data corresponding to landmarks not relevant to blinking, and only provide the facial landmark data corresponding to the landmarks relevant to blinking to the.

The training data may be assigned a total severity score and/or movement severity scores for each category of movement. In some examples, the movement severity scores of the training data can comprise a 5-point anchored score corresponding to a particular item of the AIMS. In other examples, the movement severity scores may use a different scale *(e.g.,* one to ten, A-Z, etc.). The movement severity scores of the training data may be assessed on the same or different scale as the movement severity score determined by the assessment system 104 using the trained assessment models 113a-113e, 114a-114e.The movement severity scores and/or total risk score of the training data may have been performed manually by a physician (e.g., based on recorded video data of the patient in response to the patient responding to one or more prompts). As such, the training data can comprise video data from a plurality of patients and each patient's corresponding movement severity scores and/or total severity score *(e.g.,* AIMS score) as determined by a trained clinician, including each constituent rating/answer of each score. Specifically, the training data can comprise landmarks *(e.g.,* of the face, hands, and feet) that are identified in the video data of each patient, and for instance, a movement severity score associated with the landmarks. Further, in some examples, the training data may be associated with one or more labels that characterize the video, such as a number of blinks performed by the patient during the video.

The machine learning model may include any combinations of algorithms. For example, the machine learning model may comprise gradient boosted decision trees, a random forest algorithm, a logarithmic regression model, and/or an XGBoost algorithm. In an example, gradient boosted decision trees may combine weak learners to minimize the loss function. For example, regression trees may be used to produce real values for splits and/or to be added together. The weak learners may be constrained, for example, to a maximum number of layers, a maximum number of nodes, a maximum number of splits, etc. Trees may be added one at a time to the machine learning algorithm and/or existing trees may remain unchanged. A gradient descent procedure may be used to minimize loss when adding trees. For example, additional trees may be added to reduce the loss (e.g., follow the gradient). In an example, the additional tree(s) may be given parameters and those parameters may be modified to reduce the loss. In an example, the XGBoost algorithm may comprise an implementation of gradient boosted decision trees that may be designed for speed and/or performance.

The XGBoost algorithm may (e.g., automatically) handle missing data values, support parallelization of tree construction, and/or continued training. The Gradient Boosting Trees technique may produce a prediction model in the form of an ensemble (e.g., multiple learning algorithms) of base prediction models, which are decision trees (e.g., a tree-like model of decisions and their possible consequences). The XGBoost algorithm may build a single strong learner model in an iterative fashion by using an optimization algorithm to minimize some suitable loss function (e.g., a function of the difference between estimated and true values for an instance of data). The optimization algorithm may use a training set of known values of the response variable (e.g., AIMS score) and their corresponding values of predictors (e.g., training data patient landmarks) to minimize the expected value of the loss function. The learning procedure may consecutively fit new models to provide a more accurate estimate of the response variable.

Although described primarily in the context of an XGBoost algorithm, other machine learning models may be used, such as an unsupervised learning method (e.g., a clustering method, such as a k-means or c-means clustering method) and/or a supervised learning method (e.g., gradient boosted decision trees). As an example, the assessment system 104 may use a gradient descent or a stochastic gradient descent learning method.

A supervised learning method may use labeled training data to train the machine learning algorithm. As training data is received, the supervised learning method may adjust weights until the machine learning algorithm is appropriately weighted. The supervised learning method may measure the accuracy of the machine learning algorithm using a loss function. The supervised learning method may continue adjusting the weights until the error is reduced below a predetermined threshold.

FIG. 4 is a block diagram illustrating an example computing device 400. One or more computing devices, such as the computing device 400, may implement one or more features for assessing a risk of a patient for TD, as described herein. For example, the computing device 400 may be an example of one or more of the device 106, the device 108, and/or the server device(s) 102 shown in FIG. 1A. The computing device 400 may comprise a processor 402, a memory 404, a storage device 406, an I/O interface 408, and/or a communication interface 410, which may be communicatively coupled by way of a communication infrastructure 412. It should be appreciated that the computing device 400 may include fewer or more components than those shown in FIG. 4. For instance, the computing device may include a camera (e.g., when implemented as the device 108).

The processor 402 may include hardware for executing instructions, such as those making up a computer program. In examples, to execute instructions for dynamically modifying workflows, the processor 402 may retrieve (or fetch) the instructions from an internal register, an internal cache, the memory 404, or the storage device 406 and decode and execute the instructions.

The memory 404 may be a volatile or non-volatile memory used for storing data, metadata, computer-readable or machine-readable instructions, and/or programs for execution by the processor(s) for operating as described herein. The storage device 406 may include storage, such as a hard disk, flash disk drive, or other digital storage device, for storing data or instructions for performing the methods described herein.

The memory 404 may comprise a computer-readable storage media or machine-readable storage media that maintains computer-executable instructions for performing one or more as described herein. For example, the memory 404 may comprise computer-executable instructions or machine-readable instructions that include one or more portions of the procedures described herein. The processor 402 of the device 400 may access the instructions from memory for being executed to cause the processor 402 of the device 400 to operate as described herein. The memory 404 may comprise computer-executable instructions for executing configuration software. For example, the computer-executable instructions may be executed to perform, in part and/or in their entirety, one or more procedures as described herein. Further, the memory 404 may have stored thereon one or more settings and/or control parameters associated with the device 400.

The I/O interface 408 may allow a user to provide input to, receive output from, and/or otherwise transfer data to and receive data from the computing device 400. The I/O interface 408 may include a mouse, a keypad or a keyboard, a touch screen, a camera, an optical scanner, network interface, modem, other known I/O devices or a combination of such I/O interfaces. The I/O interface 408 may include one or more devices for presenting output to a user, including, but not limited to, a graphics engine, a display *(e.g.,* a display screen), one or more output drivers *(e.g.,* display drivers), one or more audio speakers, and one or more audio drivers. The I/O interface 408 may be configured to provide graphical data to a display for presentation to a user. The graphical data may be representative of one or more graphical user interfaces and/or any other graphical content *(e.g.,* any combination of the prompts described herein).

The communication interface 410 may include hardware, software, or both. In any event, the communication interface 410 may provide one or more interfaces for communication (such as, for example, packet-based communication) between the computing device 400 and one or more other computing devices or networks. The communication may be a wired or wireless communication. As an example, and not by way of limitation, the communication interface 410 may include a network interface controller (NIC) or network adapter for communicating with an Ethernet or other wire-based network or a wireless NIC (WNIC) or wireless adapter for communicating with a wireless network, such as a WI-FI.

Additionally, the communication interface 410 may facilitate communications with various types of wired or wireless networks. The communication interface 410 may also facilitate communications using various communication protocols. The communication infrastructure 412 may also include hardware, software, or both that couples components of the computing device 400 to each other. For example, the communication interface 410 may use one or more networks and/or protocols to enable a plurality of computing devices connected by a particular infrastructure to communicate with each other to perform one or more aspects of the processes described herein.

In addition to what has been described herein, the methods and systems may also be implemented in a computer program(s), software, or firmware incorporated in one or more computer-readable media for execution by a computer(s) or processor(s), for example. Examples of computer-readable media include electronic signals (transmitted over wired or wireless connections) and tangible/non-transitory computer-readable storage media. Examples of tangible/non-transitory computer-readable storage media include, but are not limited to, a read only memory (ROM), a random-access memory (RAM), removable disks, and optical media such as CD-ROM disks, and digital versatile disks (DVDs).

While this disclosure has been described in terms of certain embodiments and generally associated methods, alterations and permutations of the embodiments and methods will be apparent to those skilled in the art. Accordingly, the above description of example embodiments does not constrain this disclosure. Other changes, substitutions, and alterations are also possible without departing from the spirit and scope of this disclosure.

## Claims

1. A computer-implemented method for assessing severity of involuntary movement associated with tardive dyskinesia (TD), the method comprising:
receiving video data (112) of a patient, wherein the video data corresponds to facial movements of the patient;
processing the video data to identify facial landmark data (118) of the patient by applying an image processing technique to the video data, wherein the image processing technique identifies facial landmarks (117) on one or more frames of the video data to produce labeled frames;
applying a plurality of trained machine learning models to the facial landmark data (118) to determine a plurality of movement severity scores (122) based on the facial landmark data of the patient, wherein each of the plurality of movement severity scores (122) is representative of a respective category of facial movement, and wherein the categories of facial movement comprise one or more of upper face movement, tongue movement, mouth and lower face movement, head shaking, or head tilting; and
processing the plurality of movement severity scores to generate a total severity score.

2. The method of claim 1, wherein a movement severity score (122) of the plurality of movement severity scores indicates a severity of involuntary movement associated with the respective category of facial movement.

3. The method of claim 1, wherein processing the video data (112) further comprises extracting and analyzing the video data to generate a facial mask comprising a plurality of uniquely labeled facial landmarks (117).

4. The method of claim 1, wherein the image processing technique comprises a dlib facial recognition method, a Haar Cascade method, a Fisherface method, or an elastic graph matching method.

5. The method of claim 4, wherein processing the video data (112) further comprises identifying the facial landmark data that corresponds to each of the facial landmarks (117) from the labeled frames, wherein the facial landmark data comprises one or more of a landmark identifier, landmark coordinate position, frame source identifier, video source identifier, or patient identifier.

6. The method of claim 5, wherein processing the video data (112) further comprises segmenting the video data into a plurality of data subsets comprising video segments of a predefined duration.

7. The method of claim 6, wherein each of the data subsets comprises a video segment that overlaps with at least one other video segment.

8. The method of claim 6, wherein applying the plurality of trained machine learning models further comprises:
applying a first trained machine learning model to at least a portion of the facial landmark data (118) of each of the data subsets to generate a movement indicator for each of the data subsets; and
applying a second trained machine learning model to the movement indicators for each of the data subsets to generate a respective movement severity score (122) for each respective category of facial movement.

9. The method of claim 8, wherein the movement indicator for each of the data subsets is a binary indicator that indicates whether the respective category of facial movement was present within the data subset.

10. The method of claim 9, wherein the first trained machine learning model is applied to a portion of the facial landmark data (118) predetermined as relevant to the respective category of facial movement of each of the data subsets.

11. The method of claim 1, wherein the plurality of movement severity scores (122) comprises a first movement severity score associated with a first category of facial movement and a second movement severity score associated with a second category of facial movement that is different from the first category of facial movement, wherein the total severity score is based on the first movement severity score and the second movement severity score.

12. The method of claim 1, wherein the total severity score corresponds to a risk of receiving a positive TD diagnosis by a trained physician.

13. The method of claim 1, wherein the plurality of trained machine learning models are trained using training data comprising facial landmark data extracted from videos from a plurality of patients with a positive TD diagnosis, Abnormal Involuntary Movement Scale (AIMS) scores associated with each patient of the plurality of patients, and labels corresponding to each of the videos indicating a presence of a plurality of distinct categories of facial movement.

14. The method of claim 1, further comprising generating a notification indicating the total severity score.

15. The method of claim 1, wherein the total severity score corresponds to an Abnormal Involuntary Movement Scale (AIMS) score.

## Patentansprüche

1. Computerimplementiertes Verfahren zum Bewerten der Schwere von unfreiwilligen Bewegungen in Zusammenhang mit Spätdyskinesie (TD), das Verfahren umfassend:
Empfangen von Videodaten (112) eines Patienten, wobei die Videodaten Gesichtsbewegungen des Patienten entsprechen;
Verarbeiten der Videodaten, um Gesichtsbezugspunktdaten (118) des Patienten durch Anwenden einer Bildverarbeitungstechnik auf die Videodaten zu identifizieren, wobei die Bildverarbeitungstechnik Gesichtsbezugspunkte (117) in einem oder mehreren Einzelbildern der Videodaten identifiziert, um markierte Einzelbilder zu erzeugen;
Anwenden einer Vielzahl von trainierten Maschinenlernmodellen auf die Gesichtsbezugspunktdaten (118), um eine Vielzahl von Bewegungsschwerebewertungen (122) zu bestimmen, basierend auf den Gesichtsbezugspunktdaten des Patienten, wobei jede der Vielzahl von Bewegungsschwerebewertungen (122) eine jeweilige Kategorie von Gesichtsbewegungen darstellt, und wobei die Kategorien von Gesichtsbewegungen eine oder mehrere von Bewegung des oberen Gesichts, Zungenbewegung, Bewegung des Mundes und des unteren Gesichts, Kopfschütteln oder Kopfneigen umfassen; und
Verarbeiten der Vielzahl von Bewegungsschwerebewertungen, um eine Gesamtschwerebewertung zu erzeugen.

2. Verfahren nach Anspruch 1, wobei eine Bewegungsschwerebewertung (122) der Vielzahl von Bewegungsschwerebewertungen eine Schwere der unfreiwilligen Bewegungen im Zusammenhang mit der jeweiligen Kategorie von Gesichtsbewegungen angibt.

3. Verfahren nach Anspruch 1, wobei die Verarbeitung der Videodaten (112) ferner das Extrahieren und Analysieren der Videodaten umfasst, um eine Gesichtsmaske zu erzeugen, die eine Vielzahl einzigartig markierter Gesichtsbezugspunkte (117) umfasst.

4. Verfahren nach Anspruch 1, wobei die Bildverarbeitungstechnik ein Dlib-Gesichtserkennungsverfahren, ein Haar-Cascade-Verfahren, ein Fisherface-Verfahren oder ein Elastic-Graph-Matching-Verfahren umfasst.

5. Verfahren nach Anspruch 4, wobei die Verarbeitung der Videodaten (112) ferner das Identifizieren der Gesichtsbezugspunktdaten, die jedem der Gesichtsbezugspunkte (117) entsprechen, aus den markierten Einzelbildern umfasst, wobei die Gesichtsbezugspunktdaten ein oder mehrere Bezugspunktidentifikatoren, Bezugspunktkoordinatenpositionen, Einzelbildquellenidentifikatoren, Videoquellenidentifikatoren oder Patientenidentifikatoren umfassen.

6. Verfahren nach Anspruch 5, wobei die Verarbeitung der Videodaten (112) ferner das Segmentieren der Videodaten in eine Vielzahl von Datenteilmengen umfasst, die Videosegmente einer vorgegebenen Dauer umfassen.

7. Verfahren nach Anspruch 6, wobei jede der Datenteilmengen ein Videosegment umfasst, das mit mindestens einem weiteren Videosegment überlappt.

8. Verfahren nach Anspruch 6, wobei das Anwenden der Vielzahl trainierter Maschinenlernmodelle ferner umfasst:
Anwenden eines ersten trainierten Maschinenlernmodells auf mindestens einen Teil der Gesichtsbezugspunktdaten (118) jeder der Datenteilmengen, um einen Bewegungsindikator für jede der Datenteilmengen zu erzeugen; und
Anwenden eines zweiten trainierten Maschinenlernmodells auf die Bewegungsindikatoren für jede der Datenteilmengen, um eine jeweilige Bewegungsschwerebewertung (122) für jede entsprechende Kategorie von Gesichtsbewegungen zu erzeugen.

9. Verfahren nach Anspruch 8, wobei der Bewegungsindikator für jede der Datenteilmengen ein binärer Indikator ist, der angibt, ob die jeweilige Kategorie von Gesichtsbewegungen in der Datenteilmenge vorhanden war.

10. Verfahren nach Anspruch 9, wobei das erste trainierte Maschinenlernmodell auf einen Teil der Gesichtsbezugspunktdaten (118) angewendet wird, der als relevant für die jeweilige Kategorie von Gesichtsbewegungen jeder der Datenteilmengen vorgegeben ist.

11. Verfahren nach Anspruch 1, wobei die Vielzahl von Bewegungsschwerebewertungen (122) eine erste Bewegungsschwerebewertung, die einer ersten Kategorie von Gesichtsbewegungen zugeordnet ist, und eine zweite Bewegungsschwerebewertung, die einer zweiten Kategorie von Gesichtsbewegungen zugeordnet ist, die sich von der ersten Kategorie von Gesichtsbewegungen unterscheidet, umfasst, wobei die Gesamtschwerebewertung auf der ersten Bewegungsschwerebewertung und der zweiten Bewegungsschwerebewertung basiert.

12. Verfahren nach Anspruch 1, wobei die Gesamtschwerebewertung einem Risiko für den Erhalt einer positiven TD-Diagnose durch einen geschulten Arzt entspricht.

13. Verfahren nach Anspruch 1, wobei die Vielzahl von trainierten Maschinenlernmodellen unter Verwendung von Trainingsdaten trainiert werden, die Gesichtsbezugspunktdaten, die aus Videos von einer Vielzahl von Patienten mit einer positiven TD-Diagnose extrahiert wurden, Bewertungen der Abnormal Involuntary Movement Scale (AIMS), die jedem Patienten der Vielzahl von Patienten zugeordnet sind, und Markierungen, die jedem der Videos entsprechen, die das Vorhandensein einer Vielzahl verschiedener Kategorien von Gesichtsbewegungen angeben, umfasst.

14. Verfahren nach Anspruch 1, ferner umfassend das Erzeugen einer Benachrichtigung, die die Gesamtschwerebewertung angibt.

15. Verfahren nach Anspruch 1, wobei die Gesamtschwerebewertung der Bewertung der Abnormal Involuntary Movement Scale (AIMS) entspricht.

## Revendications

1. Procédé mis en œuvre par ordinateur pour évaluer la sévérité des mouvements involontaires associés à la dyskinésie tardive (DT), le procédé comprenant :
la réception de données vidéo (112) provenant d'un patient, dans lequel les données vidéo correspondent aux mouvements faciaux du patient ;
le traitement des données vidéo pour identifier des données de repères faciaux (118) du patient en appliquant une technique de traitement d'image aux données vidéo, la technique de traitement d'image identifiant des repères faciaux (117) sur une ou plusieurs images des données vidéo afin de produire des images étiquetées :
l'application d'une pluralité de modèles d'apprentissage automatique entraînés aux données de repères faciaux (118) pour déterminer une pluralité de scores de sévérité des mouvements (122) sur la base des données de repères faciaux du patient, dans lequel chacun des scores de sévérité des mouvements (122) est représentatif d'une catégorie respective de mouvement facial, et dans lequel les catégories de mouvements faciaux comprennent un ou plusieurs des mouvements suivants : le mouvement de la partie supérieure du visage, le mouvement de la langue, le mouvement de la bouche et de la partie inférieure du visage, les secousses de la tête ou l'inclinaison de la tête ; et
le traitement de la pluralité de scores de sévérité des mouvements pour générer un score de sévérité total.

2. Procédé selon la revendication 1, dans lequel un score de sévérité des mouvements (122) parmi la pluralité de scores de sévérité des mouvements indique une sévérité de mouvement involontaire associée à la catégorie respective de mouvement facial.

3. Procédé selon la revendication 1, dans lequel le traitement des données vidéo (112) comprend en outre l'extraction et l'analyse des données vidéo pour générer un masque facial comprenant une pluralité de repères faciaux étiquetés de manière unique (117).

4. Procédé selon la revendication 1, dans lequel la technique de traitement d'image comprend une méthode de reconnaissance faciale ad lib, une méthode en cascade de Haar, une méthode Fisherface ou une méthode de correspondance de graphes élastiques.

5. Procédé selon la revendication 4, dans lequel le traitement des données vidéo (112) comprend en outre l'identification des données de repères faciaux qui correspondent à chacun des repères faciaux (117) à partir des images étiquetées, dans lequel les données de repères faciaux comprennent un ou plusieurs des éléments suivants : un identifiant de repère, une position de coordonnées de repère, un identifiant de source d'image, un identifiant de source vidéo ou un identifiant de patient.

6. Procédé selon la revendication 5, dans lequel le traitement des données vidéo (112) comprend en outre la segmentation des données vidéo en une pluralité de sous-ensembles de données comprenant des segments vidéo d'une durée prédéfinie.

7. Procédé selon la revendication 6, dans lequel chacun des sous-ensembles de données comprend un segment vidéo qui chevauche au moins un autre segment vidéo.

8. Procédé selon la revendication 6, dans lequel l'application de la pluralité de modèles d'apprentissage automatique entraînés comprend en outre :
l'application d'un premier modèle d'apprentissage automatique entraîné à au moins une partie des données de repères faciaux (118) de chacun des sous-ensembles de données afin de générer un indicateur de mouvement pour chacun des sous-ensembles de données ; et
l'application d'un deuxième modèle d'apprentissage automatique entraîné aux indicateurs de mouvement pour chacun des sous-ensembles de données afin de générer un score de sévérité des mouvements respectif (122) pour chaque catégorie respective de mouvement facial.

9. Procédé selon la revendication 8, dans lequel l'indicateur de mouvement pour chacun des sous-ensembles de données est un indicateur binaire qui indique si la catégorie respective de mouvement facial était présente au sein du sous-ensemble de données.

10. Procédé selon la revendication 9, dans lequel le premier modèle d'apprentissage automatique entraîné est appliqué à une partie des données de repères faciaux (118) prédéterminée comme étant pertinente pour la catégorie respective de mouvement facial de chacun des sous-ensembles de données.

11. Procédé selon la revendication 1, dans lequel la pluralité de scores de sévérité des mouvements (122) comprend un premier score de sévérité des mouvements associé à une première catégorie de mouvements faciaux et un deuxième score de sévérité des mouvements associé à une deuxième catégorie de mouvements faciaux qui est différente de la première catégorie de mouvements faciaux, dans lequel le score de sévérité total est basé sur le premier score de sévérité des mouvements et le deuxième score de sévérité des mouvements.

12. Procédé selon la revendication 1, dans lequel le score de sévérité total correspond à un risque de recevoir un diagnostic positif de DT par un médecin qualifié.

13. Procédé selon la revendication 1, dans lequel la pluralité de modèles d'apprentissage automatique entraînés sont entraînés à l'aide de données d'entraînement comprenant des données de repères faciaux extraites de vidéos provenant d'une pluralité de patients ayant reçu un diagnostic positif de DT, des scores de l'échelle des mouvements involontaires anormaux (AIMS) associés à chacun des patients de la pluralité de patients, et des étiquettes correspondant à chacune des vidéos indiquant la présence d'une pluralité de catégories distinctes de mouvements faciaux.

14. Procédé selon la revendication 1, comprenant en outre la génération d'une notification indiquant le score de sévérité total.

15. Procédé selon la revendication 1, dans lequel le score de sévérité total correspond à un score de l'échelle des mouvements involontaires anormaux (AIMS).
